(19) Europäisches Patentamt
European Patent Office
Office européen des brevets

(11) Numéro de publication: **0 252 810 B1**

(12) **FASCICULE DE BREVET EUROPEEN**

(45) Date de publication de fascicule du brevet: **17.04.91**

(51) Int. Cl.⁵: **C07C 323/39**, A61K 31/27, A61K 31/17

(21) Numéro de dépôt: **87401523.3**

(22) Date de dépôt: **01.07.87**

# (54) [Diméthylamino)-2 éthyl] [[[(méthylthio)-2-phényl] (phénylméthyl)amino]-2 oxo-2 éthyl] carbamates ou urées, leur préparation et leur application en thérapeutique.

(30) Priorité: **08.07.86 FR 8609887**

(43) Date de publication de la demande: **13.01.88 Bulletin 88/02**

(45) Mention de la délivrance du brevet: **17.04.91 Bulletin 91/16**

(84) Etats contractants désignés: **AT BE CH DE ES FR GB GR IT LI LU NL SE**

(56) Documents cités:

**Néant**

(73) Titulaire: **SYNTHELABO**
**58 rue de la Glacière**
**F-75013 Paris(FR)**

(72) Inventeur: **Beeley, Nigel**
**19, rue Cassiopée**
**F-77380 Combs La Ville(FR)**
Inventeur: **Cremer, Gérard**
**35, rue des Sables**
**F-91420 Morangis(FR)**

(74) Mandataire: **Ludwig, Jacques et al**
**SYNTHELABO, Service Brevets, 22, avenue Galilée, B.P. 72**
**F-92352 Le Plessis Robinson Cédex(FR)**

**EP 0 252 810 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention a pour objet des [(Diméthylamino)-2 éthyl][[[méthylthio)-2 phényl] (phénylméthyl)-amino]-2 oxo-2 éthyl]carbamates ou urées, leur préparation et leur application en thérapeutique.

Les composés de l'invention répondent à la formule générale I

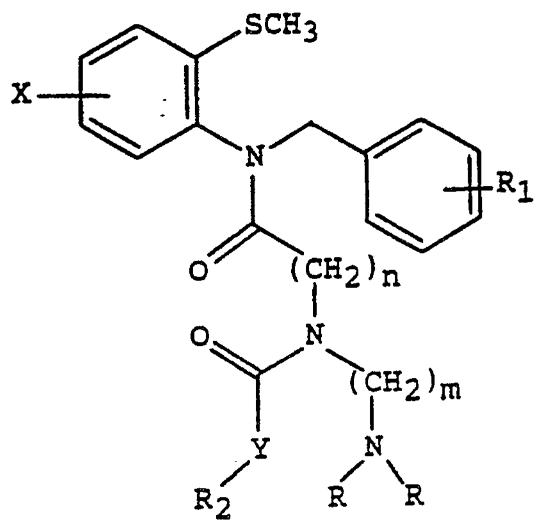

(I)

dans laquelle

X représente un atome d'hydrogène ou d'halogène ou un groupe méthylthio,

R représente un groupe alkyle en $C_1$-$C_4$,

Y représente un atome d'oxygène ou un groupe NH,

$R_1$ représente un atome d'hydrogène, un ou deux atomes d'halogène ou groupes trifluorométhyle, un groupe méthyle, un groupe méthoxy, un groupe cyano, un groupe nitro, un groupe méthoxycarbonyle, un groupe méthylènedioxy lié à deux atomes de carbone contigus du noyau benzénique qui le porte, ou encore un groupe benzo condensé formant avec le noyau benzénique qui le porte un groupe $\alpha$-naphtyle ou $\beta$-naphtyle,

$R_2$ représente un groupe alkyle ou alcényle en $C_1$-$C_8$ linéaire ou ramifié éventuellement substitué par des atomes d'halogène ou un groupe méthoxy, ou un groupe cyclopropylméthyle, ou encore un groupe phényle éventuellement substitué par un ou deux atomes d'halogène ou groupes méthyle,

n représente le nombre 1 ou 2, et

m représente le nombre 2 ou 3.

Les composés de l'invention peuvent se présenter à l'état de bases libres ou de sels d'addition à des acides acceptables en pharmacologie.

Les composés préférés sont ceux dans la formule (I) desquels n et m représentent chacun le nombre 2 et Y représente un atome d'oxygène.

On peut préparer les composés de l'invention par exemple par un procédé illustré par le schéma de la page suivante.

On condense d'abord la méthylthio-2 benzèneamine de formule II (dans laquelle X est tel que défini ci-dessus) avec un benzaldéhyde de formule III (dans laquelle $R_1$ est tel que défini ci-dessus, dans un solvant tel que le benzène, au reflux, et en présence d'acide paratoluènesulfonique, puis on effectue une hydrogénation de l'imine de formule IV ainsi obtenue, par exemple au moyen de cyanoborohydrure de sodium, dans un solvant tel que le méthanol, à température ambiante. Ensuite on fait réagir l'amine de formule V ainsi obtenue avec un chlorure d'acide de formule VI (dans laquelle n est tel que défini ci-dessus), dans un solvant tel que l'éther éthylique, à température ambiante et en présence d'une base telle que le carbonate de potassium, puis on fait réagir le chlorure de formule VII ainsi obtenu avec une diamine de formule VIII (dans laquelle R et m sont tels que définis ci-dessus), dans un solvant tel que le diméthylformamide, à température ambiante et en présence d'iodure de potassium, et enfin on fait réagir le composé de formule IX ainsi obtenu avec, lorsque Y = O, un chloroformiate de formule $R_2$-O-CO-Cl (dans laquelle $R_2$ est tel que défini ci-dessus), dans un solvant tel que l'éther éthylique, à température ambiante et en présence d'une base forte telle que la soude, ou avec, lorsque Y = NH, un isocyanate de formule $R_2$-NCO (dans laquelle $R_2$ est tel que défini ci-dessus), dans un solvant tel que le dichlorométhane, à

température ambiante.

SCHEMA

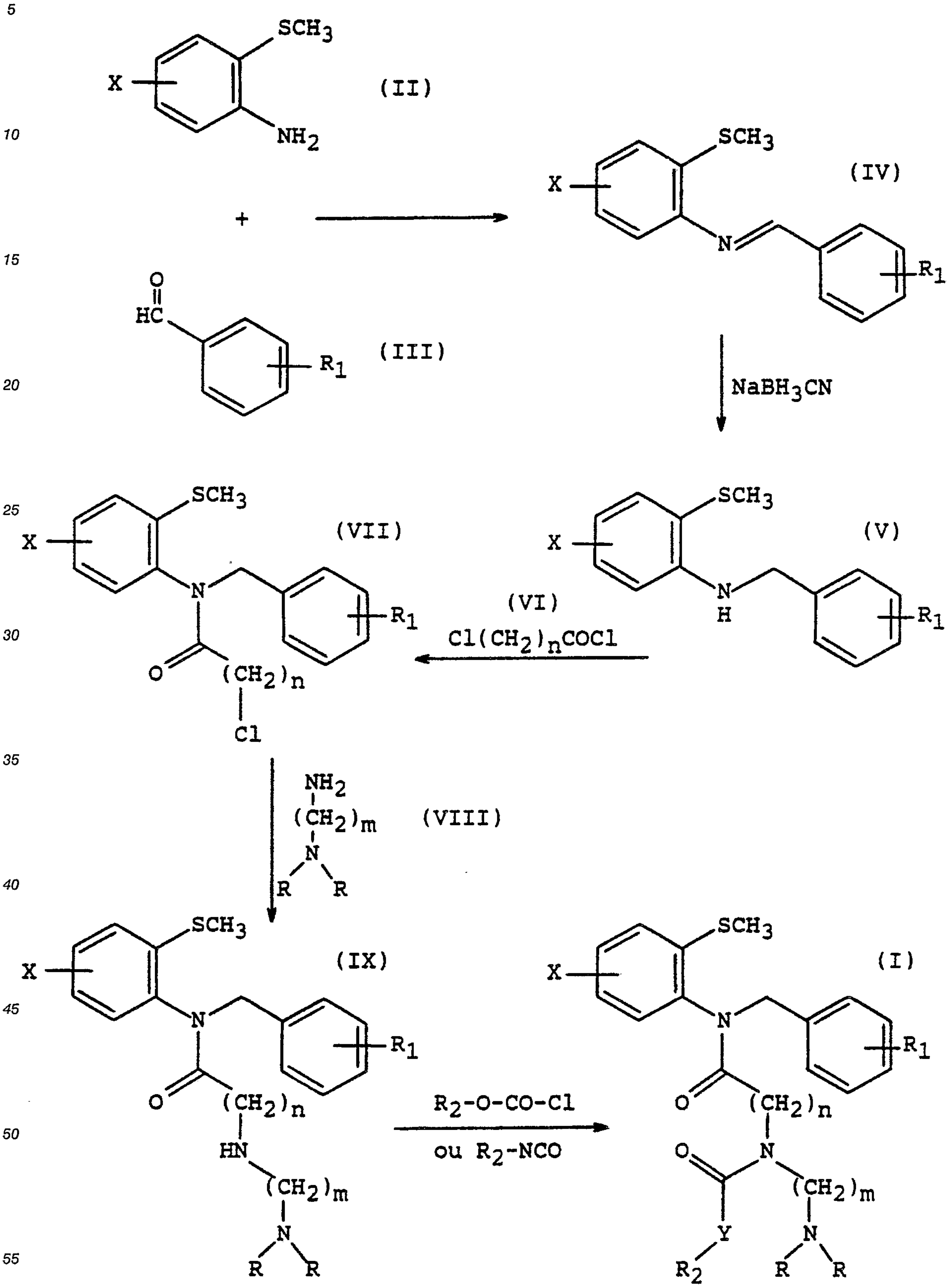
(II)
(III)
(IV)
$NaBH_3CN$
(V)
(VI)
$Cl(CH_2)_nCOCl$
(VII)
(VIII)
(IX)
$R_2$-O-CO-Cl
ou $R_2$-NCO
(I)

L'exemple ci-après illustre en détail le mode opératoire de la préparation des composés de l'invention. Le tableau qui suit illustre les structures et propriétés physique de quelques autres composés. Les microanalyses et les spectres IR et RMN ont confirmé les structures des composés ainsi décrits.Exemple.

Exemple
[(Diméthylamino)-2 éthyl][[[(méthylthio)-2 phényl][(trifluorométhyl-3 phényl)méthyl]amino-2 oxo-2 éthyl]-carbamate de méthyl-2 propyle.

1. (Méthylthio)-2 N-[[(trifluorométhyl)-3 phényl]méthylène]-benzèneamine.
On chauffe au reflux un mélange de 25 g (0,174 mole) de méthylthio-2 benzèneamine et 25,06 ml (0,174 mole) de trifluorométhyl-3 benzaldéhyde dans 250 ml de benzène en présence de 0,3 g d'acide paratoluènesulfonique, pendant 1 heure, en éliminant l'eau formée au moyen d'un appareil de Dean-Stark.
On évapore le benzène, et on utilise le résidu huileux tel quel dans l'étape suivante.
2. (Méthylthio)-2 N-[[(trifluorméthyl)-3 phényl]méthyl]-benzèneamine.
On dissout le résidu de l'étape précédente dans 300 ml de méthanol et, tout en agitant,on ajoute peu à peu 13,15 g de cyanoborohydrure de sodium. Lorsque la réaction est terminée on ajuste le pH à 6 avec de l'acide acétique, puis on chasse le solvant.
On reprend le résidu avec de l'ether et du bicarbonate de sodium, on sépare la phase organique, on la sèche et on l'évapore. On distille le résidu à 135 °C sous environ 13 Pa. On recueille 35,7 g d'amine.
3. Chloro-2 N-[(méthylthio)-2 phényl] N-[[(trifluorométhyl)-3 phényl]méthyl]acétamide.
On agite à température ambiante, pendant 5 heures, un mélange de 10 g (0,0337 mole) de l'amine précédemment préparée, 5,35 ml (0,067 mole) de chlorure de chloroacétyle et 40 g de carbonate de potassium dans 250 ml d'éther.
On sépare les produits minéraux par filtration, on lave le filtrat avec du bicarbonate de sodium puis avec de l'eau, on sèche la phase organique et on l'évapore.
Il reste 12,57 g de résidu qu'on utilise tel quel dans l'étape suivante.
4. [(Diméthylamino)-2 éthyl]amino-2 N-[(méthylthio)-2 phényl]-N-[[(trifluorométhyl)-3 phényl]méthyl]-acétamide.
On dissout le résidu de l'étape précédente dans 150 ml de diméthylformamide, on ajoute 11,07 ml (0,1 mole) de N,N-diméthyl-éthylènediamine-1,2 et une spatule d'iodure de potassium.
On agite le mélange à température ambiante pendant une nuit, puis on évapore le solvant, on reprend le résidu par du bicarbonate de sodium et de l'éther, on sépare la phase éthérée, on la sèche et on l'évapore. Il reste 12,5 g de produit brut qu'on utilise tel quel dans l'étape suivante.
5. [(Diméthylamino)-2 éthyl][[[(méthylthio)-2 phényl][(trifluorométhyl-3 phényl)méthyl]amino]-2 oxo-2 éthyl]carbamate de méthyl-2 propyle.
On dissout 3 g (0,007 mole) du produit de l'étape précédente dans 50 ml d'éther, on ajoute 25 ml d'eau puis, en surveillant le pH, on ajoute de la lessive de soude jusqu'à un pH de 12,5 à 13.
Ensuite on ajoute goutte à goutte 1,8 ml (0,014 mole) de chloroformiate d'isobutyle, en maintenant le pH par addition de soude.
On poursuit l'agitation à température ambiante pendant 10 minutes, puis on sépare la phase organique, on la sèche et on l'évapore. On purifie le résidu par chromatographie sur silice en éluant avec un mélange 99/1 de méthanol et d'éther. On recueille finalement 2 g de base pure. On prépare l'oxalate en dissolvant la base et une quantité stoechiométrique d'acide dans un minimum d'éthanol, et en isolant le sel qui cristallise.
Point de fusion : 133 °C.

Tableau

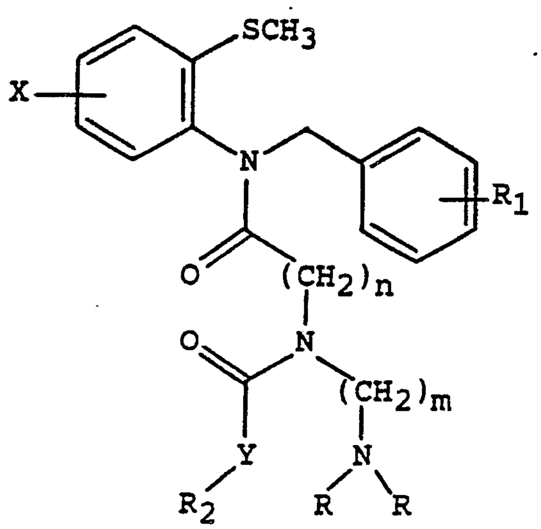

(I)

| N° | X | $R_1$ | Y | $R_2$ | R | n | m | Sel/ base | F(°C) |
|---|---|---|---|---|---|---|---|---|---|
| 1 | H | H | O | $iC_4H_9$ | $CH_3$ | 1 | 2 | 46 | 134 |
| 2 | H | 2-Cl | O | $iC_4H_9$ | $CH_3$ | 1 | 2 | 46 | 137,5 |
| 3 | H | 3-Cl | O | $iC_4H_9$ | $CH_3$ | 1 | 2 | 46 | 126 |
| 4 | H | 4-F | O | $iC_4H_9$ | $CH_3$ | 1 | 2 | 46 | 142,5 |
| 5 | H | 2,6-$Cl_2$ | O | $iC_4H_9$ | $CH_3$ | 1 | 2 | 46 | 159 |
| 6 | H | 4-$CH_3$ | O | $iC_4H_9$ | $CH_3$ | 1 | 2 | 46 | 140 |
| 7 | H | 2-CN | O | $iC_4H_9$ | $CH_3$ | 1 | 2 | 46 | 155,5 |
| 8 | H | 4-CN | O | $iC_4H_9$ | $CH_3$ | 1 | 2 | 46 | 141,5 |
| 9 | H | 2-$CF_3$ | O | $CH_3$ | $CH_3$ | 1 | 2 | 46 | 141-143 |
| 10 | H | 2-$CF_3$ | O | $iC_4H_9$ | $CH_3$ | 1 | 2 | 46 | 135-136 |
| 11 | H | 3-$CF_3$ | O | $CH_3$ | $CH_3$ | 1 | 2 | 46 | 145 |
| 12 | H | 3-$CF_3$ | O | $CH_2Cl_3$ | $CH_3$ | 1 | 2 | 46 | 122-123 |
| 13 | H | 3-$CF_3$ | O | $iC_4H_9$ | $CH_3$ | 1 | 2 | 08 | 134-135 |
| | | | | | | | | 46 | 133 |
| 14 | H | 3-$CF_3$ | O | $tC_4H_9$ | $CH_3$ | 1 | 2 | 46 | 167-169 |
| 15 | H | 3-$CF_3$ | O | $iC_5H_{11}$ | $CH_3$ | 1 | 2 | 46 | 116-118 |
| 16 | H | 3-$CF_3$ | O | $CH_2$-$tC_4H_9$ | $CH_3$ | 1 | 2 | 46 | 145-146 |
| 17 | H | 3-$CF_3$ | O | $CH(C_2H_5)_2$ | $CH_3$ | 1 | 2 | 46 | 137-138 |
| 18 | H | 3-$CF_3$ | O | $CH(CH_3)$-$iC_3H_7$ | $CH_3$ | 1 | 2 | 46 | 134-135 |
| 19 | H | 3-$CF_3$ | O | $CH_2$-$sC_4H_9$ | $CH_3$ | 1 | 2 | 46 | 118-119 |

Tableau (suite)

| N° | X | $R_1$ | Y | $R_2$ | R | n | m | Sel/ base | F(°C) |
|---|---|---|---|---|---|---|---|---|---|
| 20 | H | $3-CF_3$ | O | $CH_2-cC_3H_5$ | $CH_3$ | 1 | 2 | 46 | 125-126 |
| 21 | H | $3-OCH_2O-4$ | O | $CH_3$ | $CH_3$ | 1 | 2 | 46 | 138 |
| 22 | H | $3-OCH_2O-4$ | O | $iC_4H_9$ | $CH_3$ | 1 | 2 | 10 | 140-143 |
| 23 | H | $\alpha-C_{10}H_7$ | O | $iC_4H_9$ | $CH_3$ | 1 | 2 | 46 | 153 |
| 24 | H | $\beta-C_{10}H_7$ | O | $iC_4H_9$ | $CH_3$ | 1 | 2 | 46 | 153 |
| 25 | H | $3-CF_3$ | O | $iC_4H_9$ | $nC_3H_7$ | 1 | 2 | 46 | 78-79 |
| 26 | H | $3-OCH_2O-4$ | O | $CH_3$ | $nC_3H_7$ | 1 | 2 | 46 | 116-118 |
| 27 | H | $3-CF_3$ | O | $CH_3$ | $nC_3H_7$ | 1 | 2 | 46 | 80-82 |
| 28 | H | $3-OCH_2O-4$ | O | $iC_4H_9$ | $nC_3H_7$ | 1 | 2 | 46 | 122-124 |
| 29 | H | $3-CF_3$ | O | $iC_4H_9$ | $CH_3$ | 2 | 2 | 00 | huile |
| 30 | H | $3-CF_3$ | O | $iC_4H_9$ | $CH_3$ | 1 | 3 | 00 | huile |
| 31 | H | $3,4-Cl_2$ | O | $iC_4H_9$ | $CH_3$ | 1 | 2 | 46 | 137,5 |
| 32 | H | $2-OCH_3$ | O | $iC_4H_9$ | $CH_3$ | 1 | 2 | 46 | 128-130 |
| 33 | H | $3-CF_3$ 4-Cl | O | $iC_4H_9$ | $CH_3$ | 1 | 2 | 08 | 116 |
| 34 | H | $3-CF_3$ 6-Cl | O | $iC_4H_9$ | $CH_3$ | 1 | 2 | 08 | 123-125 |
| 35 | H | $3-CF_3$ 4-F | O | $iC_4H_9$ | $CH_3$ | 1 | 2 | 08 | 126-127 |
| 36 | H | 2-F | O | $iC_4H_9$ | $CH_3$ | 1 | 2 | 08 | 134-135 |
| 37 | H | 4-CL | O | $iC_4H_9$ | $CH_3$ | 1 | 2 | 08 | 154-155 |
| 38 | H | $2,5-Cl_2$ | O | $iC_4H_9$ | $CH_3$ | 1 | 2 | 08 | 156 |
| 39 | H | 2-Cl 6-F | O | $iC_4H_9$ | $CH_3$ | 1 | 2 | 46 | 148 |
| 40 | H | $2-NO_2$ | O | $iC_4H_9$ | $CH_3$ | 1 | 2 | 46 | 149 |
| 41 | H | $2-CH_3$ | O | $iC_4H_9$ | $CH_3$ | 1 | 2 | 46 | 123 |
| 42 | H | $4-CO_2CH_3$ | O | $iC_4H_9$ | $CH_3$ | 1 | 2 | 46 | 156-157 |
| 43 | H | $4-CF_3$ | O | $iC_4H_9$ | $CH_3$ | 1 | 2 | 08 | 134-136 |
| 44 | H | $3-CF_3$ | O | $CH(iC_3H_7)_2$ | $CH_3$ | 1 | 2 | 46 | 133-134 |
| 45 | H | $3-CF_3$ | O | $CH(iC_3H_7)(C_2H_5)$ | $CH_3$ | 1 | 2 | 46 | 129-130 |
| 46 | H | $3-CF_3$ | O | $CH(iC_4H_9)(CH_3)$ | $CH_3$ | 1 | 2 | 46 | 117-118 |
| 47 | H | $4-CF_3$ | O | $CH_2C(CH_3)_3$ | $CH_3$ | 1 | 2 | 10 | 122,5-124 |
| 48 | H | $3-CF_3$ | O | $iC_3H_7$ | $CH_3$ | 1 | 2 | 46 | 134 |
| 49 | H | $3-CF_3$ | O | $CH_2C(CH_3):CH_2$ | $CH_3$ | 1 | 2 | 46 | 128,5 |
| 50 | H | $3-CF_3$ | O | $CH_2CH:CH_2$ | $CH_3$ | 1 | 2 | 46 | 143 |
| 51 | H | $4-CF_3$ | O | $iC_3H_7$ | $CH_3$ | 1 | 2 | 46 | 142,5-143,5 |
| 52 | H | $4-CF_3$ | O | $CH_2C(CH_3):CH_2$ | $CH_3$ | 1 | 2 | 46 | 142 |
| 53 | H | $4-CF_3$ | O | $CH_2CH_2-iC_3H_7$ | $CH_3$ | 1 | 2 | 46 | Amorphe |

Tableau (suite)

| N° | X | $R_1$ | Y | $R_2$ | R | n | m | Sel/ base | F(°C) |
|---|---|---|---|---|---|---|---|---|---|
| 54 | H | 2-$OCH_3$ | O | $CH_3$ | $CH_3$ | 1 | 2 | 46 | 96-97 |
| 55 | H | 4-$CF_3$ | O | $tC_4H_9$ | $CH_3$ | 1 | 2 | 46 | 147-147,5 |
| 56 | H | 4-$CF_3$ | O | $CH_2$-$cC_3H_5$ | $CH_3$ | 1 | 2 | 46 | 137,5-139,5 |
| 57 | H | 4-$CF_3$ | O | $CH(iC_3H_7)(CH_3)$ | $CH_3$ | 1 | 2 | 46 | 142-143 |
| 58 | H | 3-$CF_3$ | O | $CH(C_2H_5)(C_5H_{11})$ | $CH_3$ | 1 | 2 | 00 | huile |
| 59 | H | 3-$CF_3$ | O | $CH(CH_3)(C_4H_9)$ | $CH_3$ | 1 | 2 | 00 | huile |
| 60 | H | 3-$CF_3$ | O | $CH_2CH_2OCH_3$ | $CH_3$ | 1 | 2 | 46 | 126,5 |
| 61 | H | 3-$CF_3$ | O | $CH(CH_3)(C_2H_5)$ | $CH_3$ | 1 | 2 | 46 | 144,5 |
| 62 | H | 3-$CF_3$ | O | $CH(C_3H_7)(iC_3H_7)$ | $CH_3$ | 1 | 2 | 46 | 131,5 |
| 63 | H | 3-$CF_3$ | O | $nC_8H_{17}$ | $CH_3$ | 1 | 2 | 00 | huile |
| 64 | 3-Cl | 4-Cl | O | $iC_4H_9$ | $CH_3$ | 1 | 2 | 46 | 86,5 |
| 65 | 4-Cl | 4-$CF_3$ | O | $iC_4H_9$ | $CH_3$ | 1 | 2 | 46 | Amorphe |
| 66 | 3-Cl | 4-F | O | $iC_4H_9$ | $CH_3$ | 1 | 2 | 46 | 120-121 |
| 67 | 3-Cl | 2-F | O | $iC_4H_9$ | $CH_3$ | 1 | 2 | 46 | 131-132 |
| 68 | 5-Cl | 4-$CF_3$ | O | $iC_4H_9$ | $CH_3$ | 1 | 2 | 46 | 144-145 |
| 69 | 6-$SCH_3$ | 4-$CF_3$ | O | $iC_4H_9$ | $CH_3$ | 1 | 2 | 46 | 152 |
| 70 | 4-Cl | 3-$CF_3$ | O | $iC_4H_9$ | $CH_3$ | 1 | 2 | 46 | 120 |
| 71 | H | 3-$CF_3$ | NH | $nC_3H_7$ | $CH_3$ | 1 | 2 | 46 | 118-119 |
| 72 | H | 3-$CF_3$ | NH | $iC_3H_7$ | $CH_3$ | 1 | 2 | 46 | 138-139 |
| 73 | H | 3-$CF_3$ | NH | $nC_4H_9$ | $CH_3$ | 1 | 2 | 46 | 126 (déc) |
| 74 | H | 3-$CF_3$ | NH | $C_6H_5$ | $CH_3$ | 1 | 2 | 46 | 132-133 |
| 75 | H | 3-$CF_3$ | NH | $C_6H_4$-F(2) | $CH_3$ | 1 | 2 | 00 | 126-127 |
| | | | | | | | | 08 | 160 (déc) |
| | | | | | | | | 46 | 162-163 |
| 76 | H | 3-$CF_3$ | NH | $C_6H_4$-F(2) | $CH_3$ | 1 | 3 | 46 | 146 |
| 77 | H | 3-$CF_3$ | NH | $C_6H_4$-F(2) | $CH_3$ | 2 | 2 | 46 | 168 |
| 78 | H | 4-$CF_3$ | NH | $C_6H_3$-$CH_3$(2)-F(5) | $CH_3$ | 1 | 2 | 46 | 143-144 |
| 79 | H | 4-$CF_3$ | NH | $C_6H_3$-$CH_3$(2)-Cl(6) | $CH_3$ | 1 | 2 | 46 | 143 |
| 80 | H | 4-$CF_3$ | NH | $C_6H_3$-$F_2$(2,6) | $CH_3$ | 1 | 2 | 46 | 171,5-172 |
| 81 | H | 4-$CF_3$ | NH | $C_6H_3$-$Cl_2$(2,6) | $CH_3$ | 1 | 2 | 46 | 159-160 |
| 82 | H | 4-$CF_3$ | NH | $C_6H_4$-F(2) | $CH_3$ | 1 | 2 | 08 | 162 |

Légende du tableau

Colonne "$R_1$" : $\alpha$-$C_{10}$-$H_7$ et $\beta$-$C_{10}H_7$ désignent des radicaux $\alpha$-naphtyle et $\beta$-naphtyle, respectivement, formés par $R_1$ et le noyau benzénique qui le porte.

Colonne "$R_2$" : $nC_3H_7$, $iC_3H_7$, $cC_3H_5$, $nC_4H_9$, $iC_4H_9$, $tC_4H_9$, $sC_4H_9$, $iC_5H_{11}$ et $nC_8H_{17}$ désignent les radicaux n-propyle, isopropyle, cyclopropyle, n-butyle, isobutyle, tertiobutyle, sec-butyle, isopentyle et n-octyle, respectivement.

$C_6H_5$, $C_6H_4$-Z(p) et $C_6H_3$-Z(p)-W(q) désignent les radicaux phényle et phényle monosubstitué ou dimbstitué par Z et/ou W aux positions p et q, respectivement.

Colonne "Sel/base" : 00, 08, 10 et 46 désignent la base libre, le fumarate, le chlorhydrate et l'oxalate, respectivement.

Les composés de l'invention ont été soumis à des essais pharmacologiques montrant leur activité en tant qu'antagonistes du calcium.

Le protocole expérimental utilisé est une variante de celui de Godfraind et Kaba (Blockade or reversal of the contraction induced by calcium and adrenaline in depolarized arterial smooth muscle, Br. J. Pharmac., (1969), 36 , 549-560).

Les expériences sont réalisées sur des tronçons d'aorte thoracique de lapin. Les animaux, des "Fauves de Bourgogne" d'un poids moyen de 1,5 kg, sont sacrifiés par dislocation cervicale et exsanguination. L'aorte thoracique est rapidement prélevée et placée dans un milieu de Krebs bicarbonaté et oxygéné (95% $O_2$ + 5% $CO_2$).

Des tronçons d'aorte de 1 cm de long environ sont préparés et installés dans des cuves à organes de 20 ml contenant de la solution de Krebs bicarbonatée et oxygénée à pH 7,4 à 37° C. Deux crochets métalliques en "U" de la longueur des tronçons sont introduits dans la lumière de ceux-ci. L'un des crochets est fixé à la base de la cuve et l'autre, relié à une jauge de contrainte isométrique (Grass FT03), permet l'enregistrement, par l'intermédiaire d'un préamplificateur continu (Grass 7P1), des réponses contractiles des tronçons d'aorte sur un oscillographe à encre (Grass 79B). Cette méthode présente, par rapport aux préparations en spirale ou en anneaux, l'avantage de mieux respecter l'intégrité structurale des vaisseaux et de n'enregistrer que la composante radiale des réponses contractiles, qui représente le phénomène intéressant du point de vue fonctionnel (régulation de la pression artérielle). Une tension initiale de 4 g est imposée aux préparations.

De la phénoxybenzamine (1 $\mu$M) et du propranolol (1 $\mu$M) sont ajoutés aux différents milieux de Krebs afin de supprimer les réponses contractiles liées à l'activation des récepteurs $\alpha$-et $\beta$-adrénergiques vasculaires.

Après une heure de stabilisation dans le milieu de Krebs, la tension imposée aux aortes est ramenée à 2 g puis, après une période d'attente de 30 minutes, les préparations sont incubées pendant une dizaine de minutes dans une solution de Krebs bicarbonatée sans calcium en présence d'EDTA (200 $\mu$M) et de propranolol (1 $\mu$M). Cette solution est alors remplacée par un milieu de Krebs dépolarisant (riche en potassium) sans calcium et contenant du propranolol (1 $\mu$M). Après 5 minutes, une concentration unique de 1 mM de calcium est ajoutée à cette solution et une période de stabilisation de 30 minutes est respectée, qui permet aux préparations d'atteindre une contraction stable.

Ensuite des doses cumulatives des composés à étudier sont administrées toutes les 30 minutes (temps généralement nécessaire pour l'obtention d'un plateau) jusqu'à disparition totale de la contraction provoquée par 1 mM de calcium ou bien jusqu'à la concentration (30 $\mu$M) de produit à étudier. En fin d'expérience une concentration supramaximale de papavérine (300 $\mu$M) est administrée afin de déterminer la décontraction maximale possible de chaque préparation.

Les valeurs absolues (en grammes) de la contraction initiale (après 1 mM de chlorure de calcium) et de la contraction après les différentes concentrations cumulatives de composés vasodilatateurs sont obtenues, pour chaque préparation, par différence avec la contraction minimale observée 30 minutes après l'addition finale de 300 $\mu$M de papavérine. Le pourcentage de diminution de la contraction, par rapport à la contraction provoquée par 1 mM de calcium, est calculé pour chaque dose de composé et chaque préparation, et la moyenne $\bar{x}$ ± E.S.M. des pourcentages individuels est calculée. Les moyennes obtenues (pondérées par l'inverse de l'Erreur Standard à la Moyenne), sont analysées à l'aide d'un modèle mathématique de courbe sigmoïde, et la concentration molaire provoquant 50% de décontraction de la réponse au calcium ($CE_{50}$) est calculée.

Pour les composés de l'invention les $CE_{50}$ vont de 0,6 $\mu$M à 30 $\mu$M.

Les composés de l'invention peuvent être utilisés pour le traitement de toutes les maladies où des antagonistes du calcium peuvent être utilisés, telles que angine de poitrine, dysrythmie cardiaque,

hypertension, cardiomyopathie, protection myocardiaque de patients à risque d'infarctus ou ayant subi un infarctus, arrêt du coeur, accidents cérébrovasculaires, manie, migraines.

D'autres essais ont révélé que les composés de l'invention sont également des inhibiteurs de l'agrégation plaquettaire, et peuvent donc être utilisés pour le traitement des affections qui en découlent.

Enfin des essais effectués sur des animaux soumis à un stress ont montré que les composés de l'invention ont encore des propriétés anti-ulcères gastriques ou gastro-duodénaux.

Les composés de l'invention peuvent être présentés sous toute forme appropriée à l'administration orale ou parentérale, en association avec tout excipient approprié, par exemple sous forme de comprimés gélules, capsules, solutions buvables ou injectables.

La posologie quotidienne peut aller de 30 à 600 mg par voie orale et de 0,06 à 180 mg par voie parentérale.

## Revendications

1. Composé de formule générale I

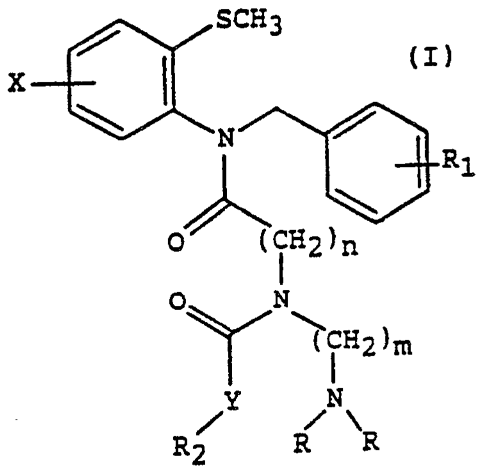

(I)

dans laquelle
X représente un atome d'hydrogène ou d'halogène ou un groupe méthylthio,
Y représente un atome d'oxygène ou un groupe NH,
R représente un groupe alkyle en $C_1$-$C_4$,
$R_1$ représente un atome d'hydrogène, un ou deux atomes d'halogène ou groupes trifluorométhyle, un groupe méthyle, un groupe méthoxy, un groupe cyano, un groupe nitro, un groupe méthoxycarbonyle, un groupe méthylènedioxy lié à deux atomes de carbone contigus du noyau benzénique qui le porte, ou encore un groupe benzo condensé formant avec le noyau benzénique qui le porte un groupe $\alpha$-naphtyle ou $\beta$-naphtyle,
$R_2$ représente un groupe alkyle ou alcényle en $C_1$-$C_8$ linéaire ou ramifié éventuellement substitué par des atomes d'halogène ou un groupe méthoxy, ou un groupe cyclopropylméthyle, ou encore un groupe phényle éventuellement substitué par un ou deux atomes d'halogène ou groupes méthyle,
n représente le nombre 1 ou 2, et
m représente le nombre 2 ou 3,
ainsi que ses sels d'additions à des acides acceptables en pharmacologie.

2. Composé selon la revendication 1, caractérisé en ce que n et m représentent chacun le nombre 2, et Y représente un atome d'oxygène.

3. Procédé de préparation d'un composé selon la revendication 1, caractérisé en ce qu'on condense d'abord une méthylthio-2 benzèneamine de formule II

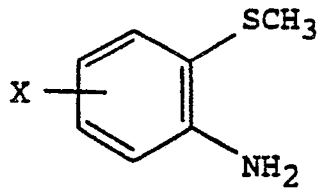

(II)

(dans laquelle X est tel que défini dans la revendication 1), avec un benzaldéhyde de formule III

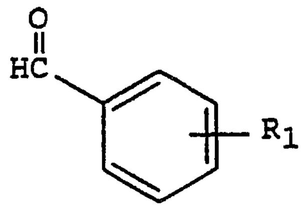

(III)

(dans laquelle $R_1$ est tel que défini dans la revendication 1), puis on effectue une hydrogénation de l'imine de formule IV ainsi obtenue

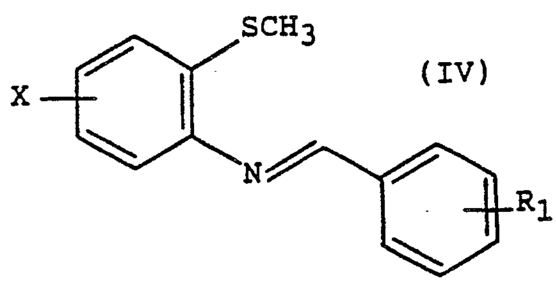

(IV)

ensuite on fait réagir l'amine de formule V ainsi obtenue

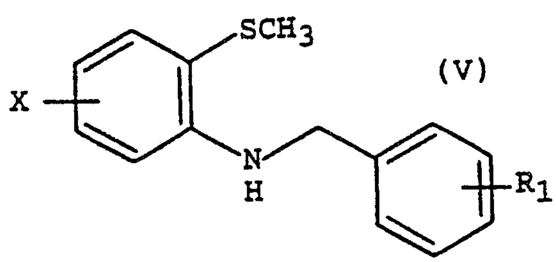

(V)

avec un chlorure d'acide de formule VI

$$Cl-(CH_2)_n-CO-Cl \qquad (VI)$$

(dans laquelle n est tel que défini dans la revendication 1), puis on fait réagir le chlorure de formule VII ainsi obtenu

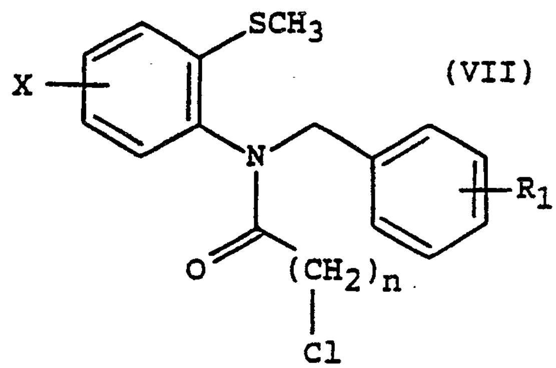

(VII)

avec une diamine de formule VIII

$$H_2N-(CH_2)_m-N(R)_2 \qquad \text{(VIII)}$$

(dans laquelle R et m sont tels que définis dans la revendication 1), et enfin on fait réagir le composé de formule IXainsi obtenu

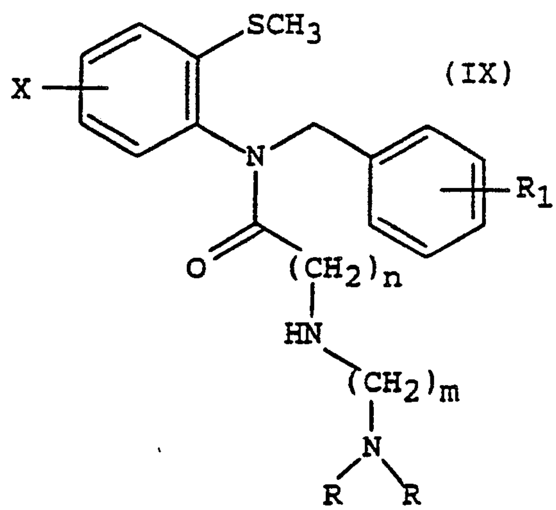

(IX)

avec un chloroformiate de formule $R_2$-O-CO-Cl ou avec un isocyanate de formule $R_2$-NCO (dans lesquelles $R_2$ est tel que défini dans la revendication 1).

4. Médicament caractérisé en ce qu'il consiste en un composé selon l'une des revendications 1 et 2.

5. Composition pharmaceutique, caractérisée en ce qu'elle contient un composé selon l'une des revendications 1 et 2, associé à un excipient acceptable en pharmacologie.

Revendication pour les Etats contractants suivants : AT, ES, GR

1. Procédé de préparation d'un composé de formule générale I

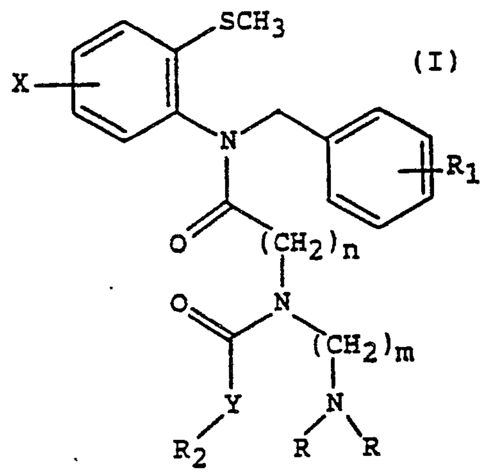

(I)

dans laquelle

X représente un atome d'hydrogène ou d'halogène ou un groupe méthylthio,

Y représente un atome d'oxygène ou un groupe NH,

R représente un groupe alkyle en $C_1$-$C_4$,

$R_1$ représente un atome d'hydrogène, un ou deux atomes d'halogène ou groupes trifluorométhyle, un groupe méthyle, un groupe méthoxy, un groupe cyano, un groupe nitro, un groupe méthoxycarbonyle, un groupe méthylènedioxy lié à deux atomes de carbone contigus du noyau benzénique qui le porte, ou encore un groupe benzo condensé formant avec le noyau benzénique qui le porte un groupe $\alpha$-naphtyle ou $\beta$-naphtyle,

$R_2$ représente un groupe alkyle ou alcényle en $C_1$-$C_8$ linéaire ou ramifié éventuellement substitué par des atomes d'halogène ou un groupe méthoxy, ou un groupe cyclopropylméthyle, ou encore un groupe phényle éventuellement substitué par un ou deux atomes d'halogène ou groupes méthyle,

n représente le nombre 1 ou 2, et

m représente le nombre 2 ou 3,

procédé caractérisé en ce qu'on condense d'abord une méthylthio-2 benzènemaine de formule II

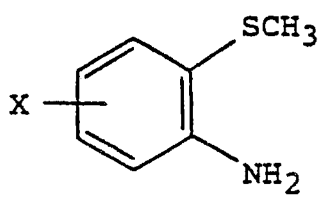

(II)

(dans laquelle X est tel que défini ci-dessus), avec un benzaldéhyde de formule III

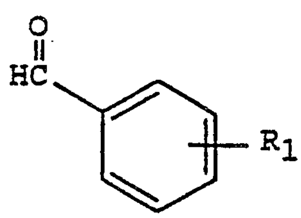

(III)

(dans laquelle $R_1$ est tel que défini ci-dessus), puis on effectue une hydrogénation de l'imine de formule IV ainsi obtenue

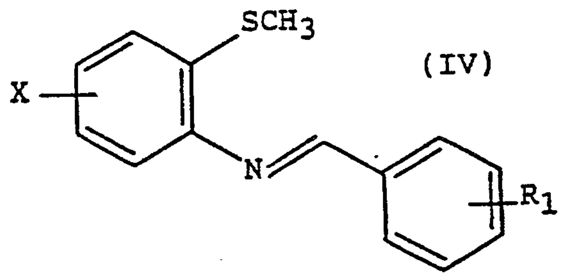

(IV)

ensuite on fait réagir l'amine de formule V ainsi obtenue

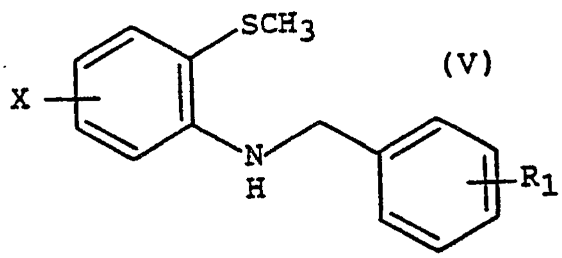

(V)

avec un chlorure d'acide de formule VI

$$Cl-(CH_2)_n-CO-Cl \qquad (VI)$$

(dans laquelle n est tel que défini ci-dessus), puis on fait réagir le chlorure de formule VII ainsi obtenu

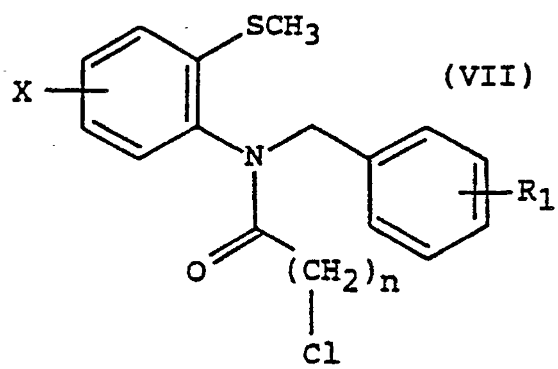

(VII)

avec une diamine de formule VIII

$$H_2N-(CH_2)_m-N(R)_2 \qquad (VIII)$$

(dans laquelle R et m sont tels que définis ci-dessus), et enfin on fait réagir le composé de formule IX ainsi obtenu

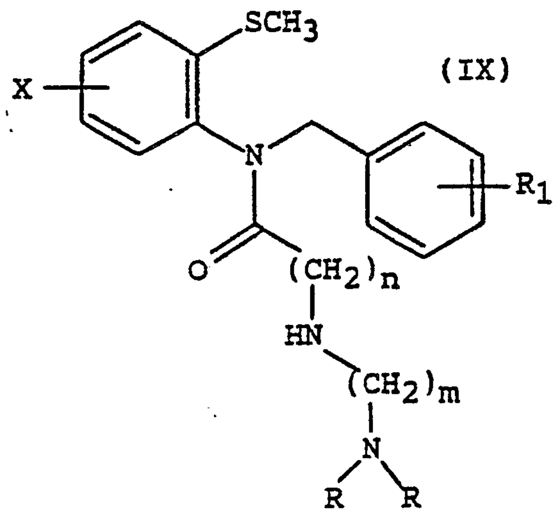

(IX)

avec un chloroformiate de formule $R_2$-O-CO-Cl ou avec un isocyanate de formule $R_2$-NCO (dans lesquelles $R_2$ est tel que défini ci-dessus).

## Claims

**1.** Compound of general formula I

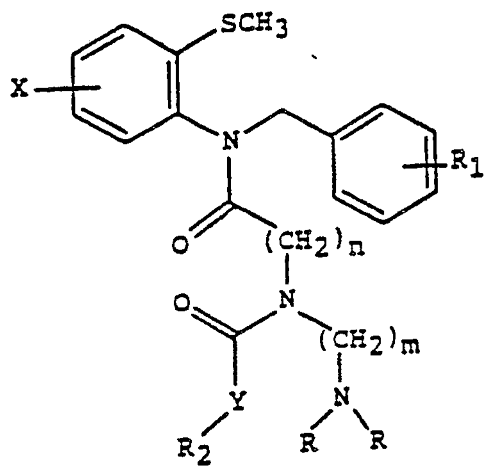

(I)

in which
X denotes a hydrogen or halogen atom or a methylthio group,
Y denotes an oxygen atom or an NH group,
R denotes a $C_1$-$C_4$ alkyl group,
$R_1$ denotes a hydrogen atom, one or two halogen atoms or trifluoromethyl groups, a methyl group, a methoxy group, a cyano group, a nitro group, a methoxycarbonyl group, a methylenedioxy group linked to two adjacent carbon atoms of the benzene ring to which it is attached, or alternatively a fused benzo group forming an α-naphthyl or β-napththyl group with the benzene ring to which it is attached,
$R_2$ denotes a linear or branched $C_1$-$C_8$ alkyl or alkenyl group optionally substituted with halogen atoms or a methoxy group, or a cyclopropylmethyl group, or alternatively a phenyl group optionally substituted with one or two halogen atoms or methyl groups,
n denotes the number 1 or 2, and
m denotes the number 2 or 3,
as well as its addition salts with pharmacologically acceptable acids.

2. Compound according to Claim 1, characterized in that n and m each denote the number 2, and Y denotes an oxygen atom.

3. Process for preparing a compound according to Claim 1, characterized in that a 2-methylthiobenzenamine of formula II

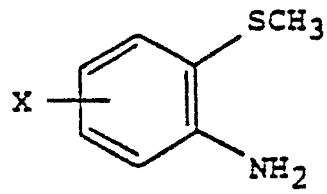

(II)

(in which X is as defined in Claim 1), is first condensed with a benzaldehyde of formula III

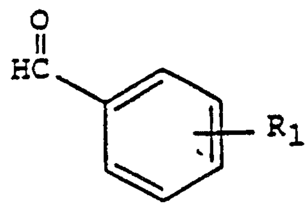

(III)

(in which $R_1$ is as defined in Claim 1), the imine of formula IV thereby obtained

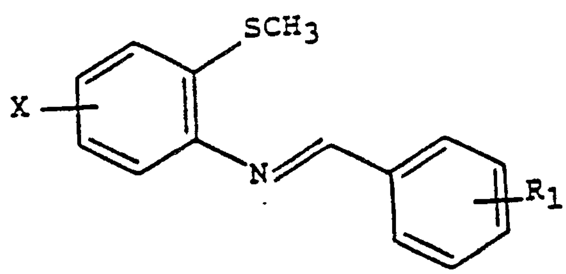

(IV)

is then hydrogenated, the amine of formula V thereby obtained

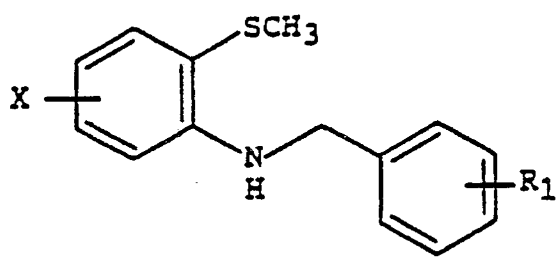

(V)

is then reacted with an acid chloride of formula VI

$$Cl-(CH_2)_n-CO-Cl \quad (VI)$$

(in which n is as defined in Claim 1), the chloride of formula VII thereby obtained

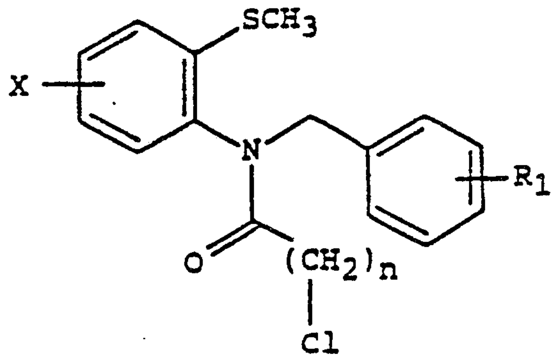

(VII)

is then reacted with a diamine of formula VIII

$$H_2N-(CH_2)_m-N(R)_2 \qquad (VIII)$$

(in which R and m are as defined in Claim 1), and finally the compcund of formula IX thereby obtained

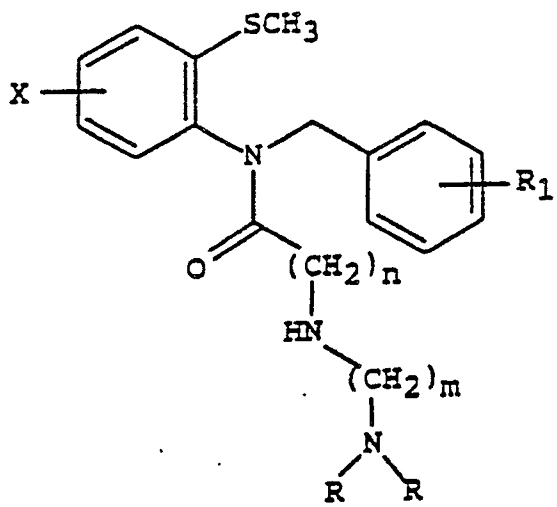

(IX)

is reacted with a chloroformate of formula $R_2$-O-CO-Cl or with an isocyanate of formula $R_2$-NCO (in which formulae $R_2$ is as defined in Claim 1).

4. Medicinal product, characterized in that it consists of a compound according to one of Claims 1 and 2.

5. Pharmaceutical composition, characterized in that it contains a compound according to one of Claims 1 and 2, in combination with a pharmacologically acceptable excipient.

Claim for the following Contracting States : AT, ES, GR

1. Process for preparing a compound of general formula I

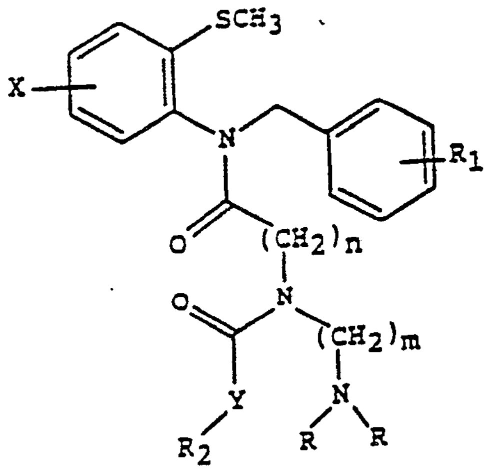

(I)

in which

X denotes a hydrogen or halogen atom or a methylthio group,

Y denotes an oxygen atom or an NH group,

R denotes a $C_1$-$C_4$ alkyl group,

$R_1$ denotes a hydrogen atom, one or two halogen atoms or trifluoromethyl groups, a methyl group, a methoxy group, a cyano group, a nitro group, a methoxycarbonyl group, a methylenedioxy group linked to two adjacent carbon atoms of the benzene ring to which it is attached, or alternatively a fused benzo group forming an $\alpha$-naphthyl or $\beta$-napththyl group with the benzene ring to which it is attached,

$R_2$ denotes a linear or branched $C_1$-$C_8$ alkyl or alkenyl group optionally substituted with halogen atoms or a methoxy group, or a cyclopropylmethyl group, or alternatively a phenyl group optionally substituted with one or two halogen atoms or methyl groups,

n denotes the number 1 or 2, and

m denotes the number 2 or 3,

which process is characterized in that a 2-methylthiobenzenamine of formula II

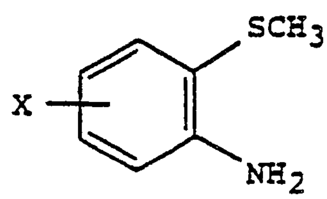

(II)

(in which X is as defined above), is first condensed with a benzaldehyde of formula III

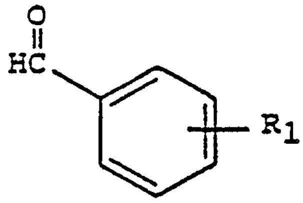

(III)

(in which $R_1$ is as defined above), the imine of formula IV thereby obtained

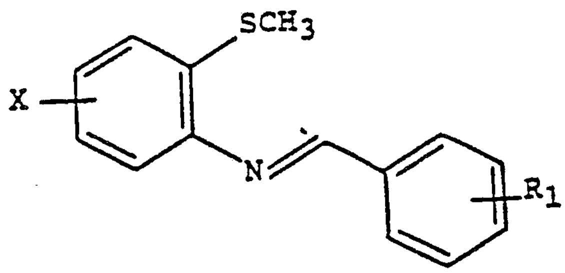

(IV)

is then hydrogenated, the amine of formula V thereby obtained

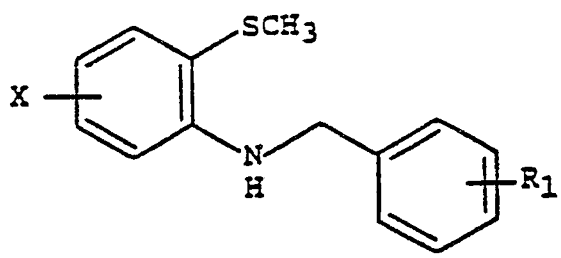

(V)

is then reacted with an acid chloride of formula VI

$$Cl-(CH_2)_n-CO-Cl \quad (VI)$$

(in which n is as defined above), the chloride of formula VII thereby obtained

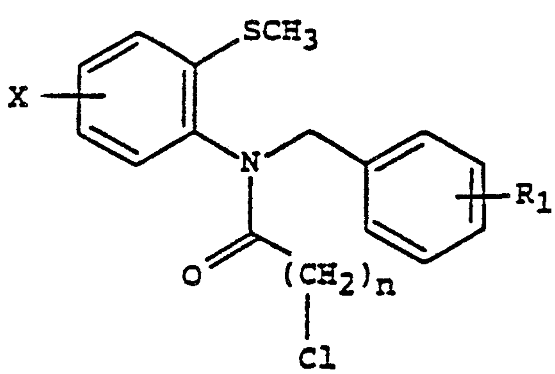

(VII)

is then reacted with a diamine of formula VIII

$$H_2N-(CH_2)_m-N(R)_2 \quad (VIII)$$

(in which R and m are as defined above), and finally the compound of formula IX thereby obtained

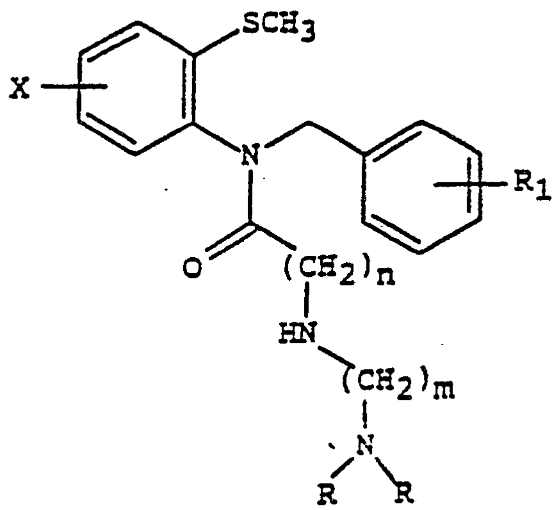

(IX)

is reacted with a chloroformate of formula $R_2$-O-CO-Cl or with an isocyanate of formula $R_2$-NCO (in which formulae $R_2$ is as defined above).

## Ansprüche

1. Verbindung der allgemeinen Formel I

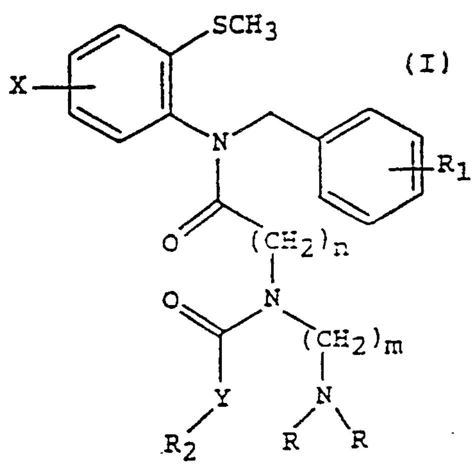

(I)

in welcher X für ein Wasserstoff- oder Halogenatom oder eine Methylthiogruppe, Y für ein Sauerstoffatom oder für die Gruppe NH und R für eine $C_1$-$C_4$-Alkylgruppe steht, $R_1$ ein Wasserstoffatom, ein oder zwei Halogenatome oder Trifluormethylgruppen, eine Methyl-, Methoxy-, Cyano-, Nitro- -oder Methoxycarbonylgruppe, eine Methylendioxygruppe, die an zwei dem sie tragenden Benzolkern angehörige Kohlenstoffatome gebunden ist, oder eine kondensierte Benzogruppe, die mit dem sie tragenden Benzolkern eine α-Naphthyl oder β-Naphthylgruppe bildet, bedeutet, $R_2$ eine lineare oder verzweigte, gegebenenfalls durch Halogenatome oder eine Methoxygruppe substituierte $C_1$-$C_8$-Alkyl- oder - Alkenylgruppe, eine Cyclopropylmethylgruppe oder eine gegebenenfalls durch ein oder zwei Halogenatome oder Methylgruppen substituierte Phenylgruppe darstellt, n für die Zahl 1 oder 2 und m für die Zahl 2 oder 3 steht, sowie deren Additionssalze mit pharmakologisch verwendbaren Säuren.

2. Verbindung nach Anspruch 1, **dadurch gekennzeichnet,** daß n und m jeweils für die Zahl 2 und Y für ein Sauerstoffatom stehen.

3. Verfahren zur Herstellung einer Verbindung nach Anspruch 1, **dadurch gekennzeichnet,** daß man zuerst ein 2-Methylthiobenzamin der Formel II

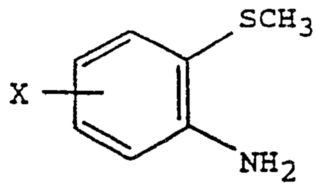

(II)

(in welcher X die in Anspruch 1 genannte Bedeutung hat), mit einem Renzaldehyd der Formel III

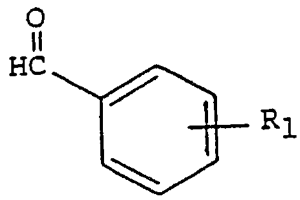

(III)

(in welcher $R_1$ die in Anspruch 1 genannte Bedeutung hat), kondensiert, anschließend eine Hydrierung des auf diese Weise erhaltenen Imins der Formel IV

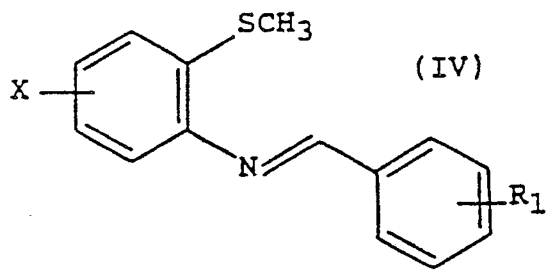

(IV)

durchführt, dann das so erhaltene Amin der Formel V

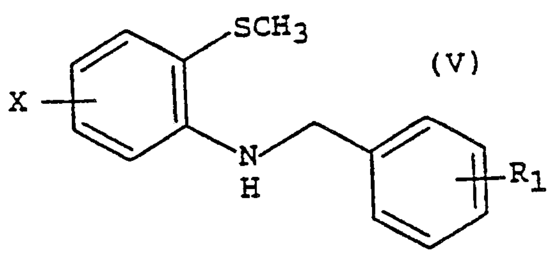

(V)

mit einem Säurechlorid der Formel VI

$$Cl-(CH_2)_n-CO-Cl \qquad (VI)$$

(in welcher n die in Anspruch 1 angegebenen Bedeutung hat), reagieren läßt, worauf man das so erhaltene Chlorid der Formel VII

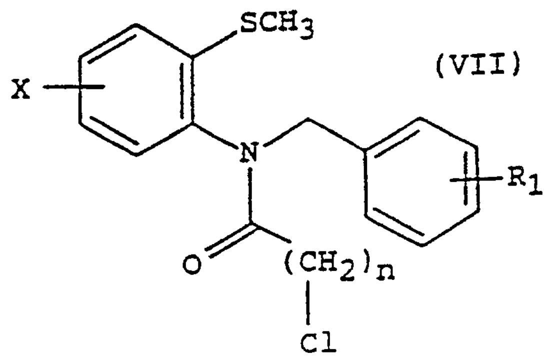

(VII)

mit einem Diamin der Formel VIII

$$H_2N-(CH_2)_m-N(R)_2 \qquad (VIII)$$

(in welcher R und m die in Anspruch 1 genannte Bedeutung haben), zur Reaktion bringt und schließlich die so erhaltene Verbindung IX

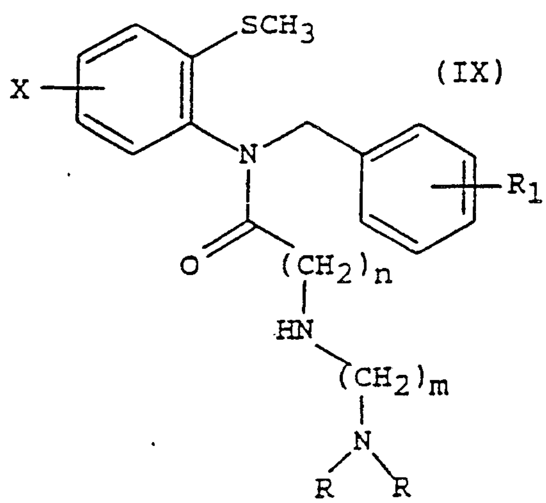

(IX)

mit einem Chloroformiat der Formel $R_2$-O-CO-Cl oder mit einem Isocyanat der Formel $R_2$-NCO (in welchen Formeln $R_2$ die in Anspruch 1 genannte Bedeutung hat), reagieren läßt.

4. Arzneimittel, **dadurch gekennzeichnet,** daß es aus einer Verbindung nach Anspruch 1 oder 2 besteht.

5. Pharmazeutische Zusammensetzung, **dadurch gekennzeichnet,** daß sie eine Verbindung nach Anspruch 1 oder 2 in Kombination mit einem pharmakologisch verwendbaren Träger enthält.

Patentanspruch für folgende Vertragsstaaten : AT, ES, GR

1. Verfahren zur Herstellung einer Verbindung der allgemeinen Formel I

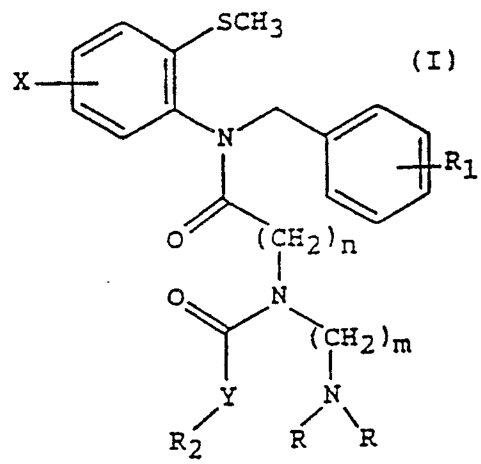

(I)

in welcher X für ein Wasserstoff- oder Halogenatom oder eine Methylthiogruppe, Y für ein Sauerstoffatom oder für die Gruppe NH und R für eine $C_1$-$C_4$-Alkylgruppe steht, $R_1$ ein Wasserstoffatom, ein oder zwei Halogenatome oder Trifluormethylgruppen, eine Methyl-, Methoxy-, Cyano-, Nitro- -oder Methoxycarbonylgruppe, eine Methylendioxygruppe, die an zwei dem sie tragenden Benzolkern angehörige Kohlenstoffatome gebunden ist, oder eine kondensierte Benzogruppe, die mit dem sie tragenden Benzolkern eine α-Naphthyl oder β-Naphthylgruppe bildet, bedeutet, $R_2$ eine lineare oder verzweigte, gegebenenfalls durch Halogenatome oder eine Methoxygruppe substituierte $C_1$-$C_8$-Alkyl- oder - Alkenylgruppe, eine Cyclopropylmethylgruppe oder eine gegebenenfalls durch ein oder zwei Halogenatome oder Methylgruppen substituierte Phenylgruppe darstellt, n für die Zahl 1 oder 2 und m für die Zahl 2 oder 3 steht, dadurch gekennzeichnet, daß man zuerst ein 2-Methylthiobenzamin der Formel II

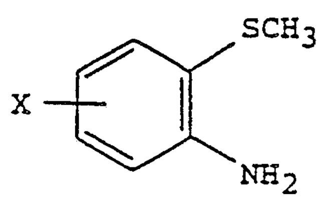

(II)

in welcher X die oben genannte Bedeutung hat), mit einem Benzaldehyd der Formel III

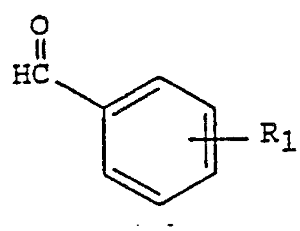

(III)

(in welcher $R_1$ die oben genannte Bedeutung hat), kondensiert, anschließend eine Hydrierung des auf diese Weise erhaltenen Imins der Formel IV

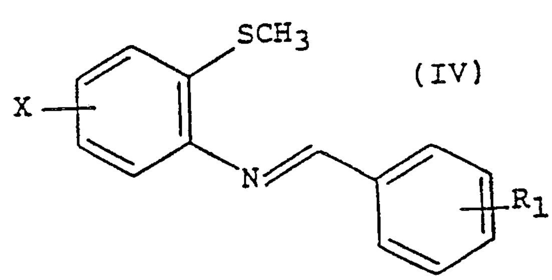

(IV)

durchführt, dann das so erhaltene Amin der Formel V

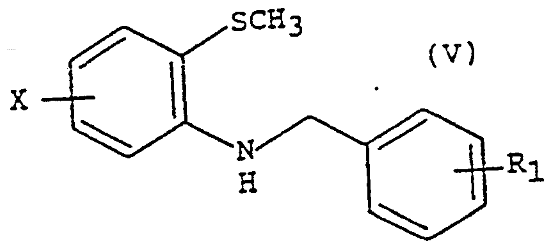

(V)

mit einem Säurechlorid der Formel VI

$$Cl-(CH_2)_n-CO-Cl \qquad (VI)$$

(in welcher n die oben angegebenen Bedeutung hat), reagieren läßt, worauf man das so erhaltene Chlorid der Formel VII

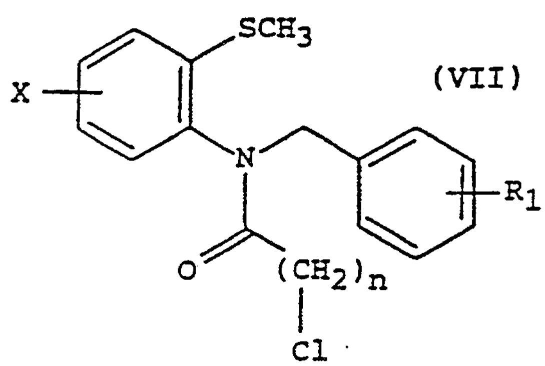

(VII)

mit einem Diamin der Formel VIII

$$H_2N-(CH_2)_m-N(R)_2 \qquad (VIII)$$

(in welcher R und m die oben genannte Bedeutung haben), zur Reaktion bringt und schließlich die so erhaltene Verbindung IX

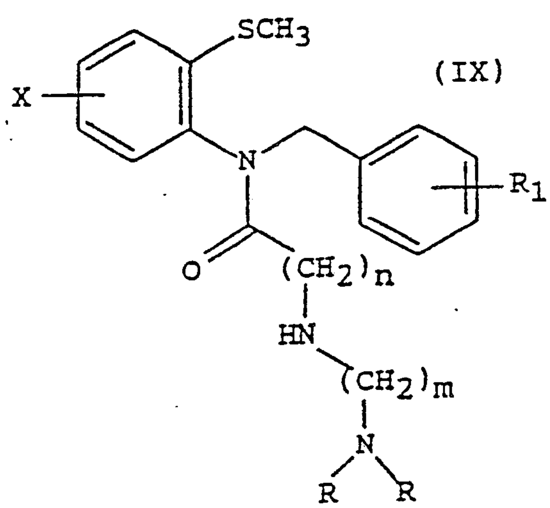

(IX)

mit einem Chloroformiat der Formel $R_2$-O-CO-Cl oder mit einem Isocyanat der Formel $R_2$-NCO (in welchen Formeln $R_2$ die oben genannte Bedeutung hat), reagieren läßt.